# EUROPÄISCHE PATENTSCHRIFT

(11) **EP 2 657 698 B1**
(45) Veröffentlichungstag und Bekanntmachung des Hinweises auf die Patenterteilung: **09.01.2019**
(21) Anmeldenummer: 13165672.0
(22) Anmeldetag: 27.04.2013
(51) Int. Cl.: G01N 33/28

(54) **Verfahren zum Ermitteln der Cetanzahl von Kraftstoffen**
Method for determining the cetane number of fuels
Procédé de détermination de l'indice de cétane de carburants

(30) Priorität: 27.04.2012 DE 102012008410
(43) Veröffentlichungstag der Anmeldung: 30.10.2013
(73) Patentinhaber: Regineering GmbH, 85095 Denkendorf (DE)
(72) Erfinder: Innerhofer, Stefan, 85080 Gaimersheim (DE)
(74) Vertreter: Oberhardt, Knut

(56) Entgegenhaltungen:
- WO-A1-2006/136349
- DE-A1-102008 001 307
- US-A1- 2009 198 456
- US-A1- 2010 268 444

## Beschreibung

Die Erfindung betrifft ein Verfahren zum Ermitteln einer Kennzahl von Kraftstoffen nach dem Oberbegriff von Anspruch 1.

Die Cetanzahl ist ein Maß für die Zündwilligkeit eines Kraftstoffes, wie er in dieselmotorischen Brennkraftmaschinen verwendet wird. Um die Cetanzahl eines Kraftstoffes zu ermitteln, wird dieser üblicherweise mit verschiedenen Prüfstoffen aus einer Mischung von Cetan und Alpha-Methyl-Naphtalin verglichen. Die Cetanzahl bezieht sich dabei auf den prozentualen Anteil von Cetan in dem entsprechenden Prüfstoff. So wird davon ausgegangen, dass ein Prüfstoff, der ausschließlich aus Cetan besteht, die Cetanzahl 100, ein Prüfstoff, der ausschließlich aus Alpha-Methyl-Naphtalin besteht, die Cetanzahl 0 und ein Prüfstoff, der beispielsweise aus 60% Cetan und 40% Alpha-Methyl-Naphtalin besteht, die Cetanzahl 60 besitzt. Verhält sich ein zu prüfender Kraftstoff in einem Prüfmotor beispielsweise genauso wie der oben angegebene Prüfstoff mit einem Cetananteil von 60%, so wird diesem Kraftstoff die Cetanzahl 60 zugeteilt.

Als Prüfmotor wird üblicherweise der sogenannte BASF-Motor eingesetzt. Bei diesem Einzylinder-Prüfmotor wird der Einspritzbeginn fest 20° Kurbelwinkel (KW) vor dem oberen Totpunkt (OT) eingestellt. Der Zündbeginn wird über den Druckanstieg im Zylinder durch einen Drucksensor ermittelt. Während des Betriebs mit dem zu prüfenden Kraftstoff wird die angesaugte Luft über eine veränderbare Drossel so verändert, dass der Zündbeginn im OT liegt. Es wird folglich über die Zufuhr der Verbrennungsluft eine Zündverzögerung von 20° KW eingestellt. Nun wird ein Prüfstoff gesucht, der bei gleicher Einstellung der Verbrennungsluft ebenfalls einen Zündverzug von 20° KW aufweist. Die Cetanzahl des passenden Prüfstoffes wird dem zu prüfenden Kraftstoff dann zugeteilt. Bei diesem Verfahren wird also die Menge der zugeführten Verbrennungsluft zur Bestimmung der Cetanzahl verwendet.

Es wurde jedoch festgestellt, dass die Zündwilligkeit eines Kraftstoffes auch von Bedingungen abhängt, wie Sie zwar in Dieselmotoren der neuen Generation vorkommen, die aber mit dem BASF-Motor nicht darstellbar sind. Es kann daher bei der Einteilung von Kraftstoffen zu Verzerrungen gegenüber dem Bild kommen, das diese Kraftstoffe beim Betrieb in modernen Dieselmotoren abgeben.

Das bekannte Verfahren mit dem BASF-Prüfmotor konnte eingesetzt werden, solange die Prüfkraftstoffe und die zu prüfenden Kraftstoffe eine ähnliche Viskosität, ähnliche Oberflächenspannung und einen ähnlichen Siedeverlauf aufwiesen. Die früher eingesetzten Kraftstoffe auf Erdölbasis waren in diesen Parametern alle sehr ähnlich.Heute unterscheiden sich Kraftstoffe jedoch sehr stark, da sie aus unterschiedlichsten Rohstoffen gewonnen und zum Teil sogar untereinander vermischt werden. Bei diesen unterschiedlichen Kraftstoffen verläuft der Zerfall des eingespritzten Kraftstoffstrahls und die Verdampfung des zerfallenen Strahls in dem BASF-Prüfmotor sehr unterschiedlich.

Es ergibt sich deshalb als weiteres Problem, dass der BASF-Prüfmotor aufgrund seines festgelegten Einspritzbeginns, seines geringen Einspritzdrucks und des damit zusammenhängenden geringen Zerfalls des Einspritzstrahls nicht mit allen heute auf dem Markt vorkommenden Kraftstoffen für dieselmotorische Brennkraftmaschinen betreibbar ist. So ist es z. B. nicht möglich, diesen Motor mit Pflanzenöl zu betreiben und die Cetanzahl von Pflanzenöl zu bestimmen.

Es wurde auch versucht, die Zündwilligkeit von Kraftstoffen in beheizten Hochdruckbehältern ohne bewegliche Kolben zu ermitteln. Bei diesen Fuel Ignition Analysern (FIA) wird Kraftstoff in eine vorgeheizte Kammer mit einem vorbestimmten Druck eingespritzt und die Zeit bis zur Selbstzündung gemessen. Der Grundgedanke bei der Entwicklung der FIAs war es, für alle Kraftstoffe absolut gleiche Bedingungen zu schaffen. Bei einer Messung nach diesem Verfahren ergibt sich somit aber nur ein theoretischer Wert, der jedoch wenig Bezug zu den Bedingungen in einem modernen Dieselmotor aufweist und daher auch wenig Relevanz hat.

So erfolgt beispielsweise die Beheizung der Hochdruckkammer eines FIA über die Kammerwände, so dass diese auf einer Temperatur gehalten werden müssen, die bei einer Zylinderwand eines Motors niemals vorkommt. Auch kann in einem FIA die Motordynamik, die durch den sich auf und ab bewegenden Zylinder zustande kommt und die damit in Verbindung stehende Druckveränderung innerhalb des Verbrennungsraums nicht nachgestellt und berücksichtigt werden. Die Strömungsbedingungen, die in einem modernen Dieselmotor herrschen, können in einem FIA ebenfalls nicht nachgebildet werden. Es kommt daher auch bei der in einem FIA ermittelten Zündwilligkeit von Kraftstoffen zu einem anderen Bild, als dasjenige, welches sich beim Betrieb moderner Dieselmotoren mit diesen Kraftstoffen ergibt.

Weitere artverwandte Verfahren zur Bestimmung von Cetanzahlen werden in US2010/268444 sowie in US2009/0198456 offenbart.

Der Erfindung liegt die Aufgabe zugrunde, ein Verfahren zur Ermittlung der Cetanzahl von Kraftstoffen zu schaffen, das an die Bedingungen in modernen Dieselmotoren angepasst ist, mit dem alle bekannten Dieselkraftstoffe reproduzierbar geprüft werden können.

Gelöst wird die Aufgabe gemäß der Erfindung durch ein Verfahren zur Ermittlung der Cetanzahl von Kraftstoffen mit den Merkmalen von Anspruch 1. Dadurch, dass für den zu prüfenden Kraftstoff der Einspritzbeginn ermittelt wird, bei dem der Prüfmotor bei unveränderter Einspritzmenge die höchste Leistung erreicht, wird die Zündverzögerung erstmals unter optimalen Verbrennungsbedingungen und nicht durch eine Drosselung der Verbrennungsluft bestimmt. Es ergibt sich dadurch auch erstmals bei der Vermessung von unterschiedlichsten Dieselkraftstoffen ein Bild, das demjenigen entspricht, das von dem Betrieb moderner Dieselmotoren mit solchen unterschiedlichen Kraftstoffen bekannt ist. Es wird auch erstmals möglich, die Cetanzahl von Kraftstoffen zu bestimmen, mit denen der BASF-Prüfmotor aufgrund seines festgelegten Einspritzbeginns und der damit verbundenen schlechten Verbrennungsbedingungen erst gar nicht betreibbar war.

Für den Einspritzbeginn sind unterschiedliche Definitionen bekannt geworden. Da es hier nur darauf ankommt, den Einspritzbeginn immer in gleicher Weise festzulegen, wird er hier als der Zeitpunkt definiert, zu dem die Motorsteuerung den Befehl zum Öffnen der Einspritzdüse absetzt. Die Einspritzmenge ist die Menge an Kraftstoff, die dem Motor pro Einspritz-Zyklus über die Einspritzdüse zugeführt wird. Die Einspritzmenge ist abhängig von dem Einspritzdruck und der Einspritzzeit, wobei der Einspritzdruck derjenige Druck ist, der an der geschlossenen Einspritzdüse ansteht und wobei die Einspritzzeit als die Öffnungszeit der Einspritzdüse pro Einspritz-Zyklus festgelegt wird.

Als Prüfstoffe werden üblicherweise Gemische aus Cetan und Alpha-Methyl-Naphtalin in verschiedenen Mischungsverhältnissen verwendet. Über das Mischungsverhältnis ist auch die Cetanzahl des jeweiligen Prüfstoffs bekannt.

Es besteht die Möglichkeit bei jedem zu prüfenden Kraftstoff zu ermitteln, bei welcher Drehzahl und bei welchem Einspritzdruck die maximale Leistung erzielt werden kann. Dieses Vorgehen würde jedoch einen enormen Zeitaufwand erfordern, da diese Prüfung für jeden eingestellten Einspritzbeginn durchgeführt werden müsste. Es wird deshalb vorteilhaft die höchste Leistung bei unverändertem Einspritzdruck, unveränderter Einspritzmenge und gleicher Drehzahl ermittelt, so dass nur der Einspritzbeginn für die Messungen verändert werden muss.

Bei unterschiedlichen Arten der Einspritzung kann es zu unterschiedlichen Ergebnissen bei der der Ermittlung des Einspritzbeginns mit der höchsten Leistung kommen. So verhält sich ein Prüfmotor mit unterteiltem Einspritzzyklus - wenn also beispielsweise mit einer Voreinspritzung gearbeitet wird - anders als ein Prüfmotor, bei dem pro Einspritzzyklus nur einmal kontinuierlich eingespritzt wird. Auch hier ist wiederum wichtig, dass der Prüfmotor immer in gleicher Weise betrieben wird, um reproduzierbare Ergebnisse zu erhalten. Bei dem erfindungsgemäßen Verfahren wird deshalb mit einer nicht unterbrochenen kontinuierlichen Einspritzung gearbeitet. Sollte sich herausstellen, dass zukünftig auch Kraftstoffe zu vermessen sind, mit denen der Prüfmotor unter Verwendung dieser Einspritzart nicht betreibbar ist. kann auch auf eine unterbrochene Einspritzung umgestellt werden. Es ist nur darauf zu achten, dass in diesem Fall auch eine Kalibrierung mit dieser Einspritzart mit den Prüfstoffen vorgenommen werden muss.

Die Prüfstoffe, die eine bekannte Cetanzahl aufweisen, müssen in der selben Weise vermessen werden wie ein zu prüfender Kraftstoff. Es wird deshalb auch bei einem Betrieb des Prüfmotors mit den Prüfstoffen für jeden Prüfstoff der Einspritzbeginn bei der höchsten erreichten Leistung ermittelt. Aus diesen Werten kann dann eine Kurve festgelegt werden, in der jeder Cetanzahl ein Einspritzbeginn zugeordnet ist. Eine solche Kalibrierung ist nur nach längeren Zeitabschnitten erforderlich.

Besonders vorteilhaft wird der Prüfmotor mit dem zu prüfenden Kraftstoff betrieben, der Einspritzbeginn jeweils nach einer Stabilisierung des Prüfmotors in vorbestimmten Schritten verändert, zu jedem eingestellten Einspritzbeginn die Leistung des Prüfmotors ermittelt und zumindest die höchste erreichte Leistung zusammen mit dem dabei eingestellten Einspritzbeginn gespeichert. Diese Vorgehensweise kann weitgehend automatisiert werden. Die Ermittlung der Cetanzahl erfolgt dann über einen Vergleich mit der Kalibrierkurve.

Nach einer Kalibrierung des Prüfmotors muss für einen längeren Zeitraum keine weitere Kalibrierung durchgeführt werden. Da ein Prüfmotor durch den Verschleiß beispielsweise der Einspritzdüse einer bestimmten Veränderung unterliegt wird erfindungsgemäß in vorbestimmten kürzeren Abständen eine Validierung des Prüfmotors durchgeführt.

Hierzu wird bei dem Betrieb mit nur einem Prüfstoff mit bekannter Cetanzahl der Einspritzbeginn ermittelt, bei dem der Prüfmotor die höchste Leistung erreicht. Die Abweichung, die sich gegenüber einer früheren Kalibrierung oder Validierung ergibt wird auf die anderen Prüfstoffe übertragen, ohne dass diese im Einzelnen neu vermessen werden müssen. Es ergibt sich auf diese Weise eine geänderte Kalibrierkurve, mit der die zu prüfenden Kraftstoffe verglichen werden.

Als Prüfmotor wird ein möglichst einfach aufgebauter Motor verwendet, der aber einem modern aufgebauten Dieselmotor entspricht. Vorteilhaft weist der Prüfmotor wenigstens eine Hochdruckpumpe, ein Commonrail, einen Zylinder und eine Einspritzdüse auf. Weiterhin ist der Prüfmotor so ausgebildet, dass der Einspritzbeginn während des Betriebs verstellbar ist. Auf diese Weise wird es möglich, eine Versuchsreihe mit unterschiedlichen Einspritzzeitpunkten durchzufahren, ohne den Prüfmotor zwischenzeitlich abstellen zu müssen.

Um die Prüfung eines Kraftstoffes weitgehend automatisieren zu können, muss der Einspritzzeitpunkt während des Betriebs des Prüfmotors programmgesteuert verstellbar sein. Es ist deshalb ein Rechner vorgesehen, der den Einspritzbeginn in bestimmten Schritten verstellt. Die Zeitabstände sind so gewählt, dass sich der Lauf des Prüfmotors nach jeder Verstellung stabilisieren kann. Sobald eine Stabilisierung eingetreten ist, erfolgt vor jeder neuen Verstellung eine Leistungsmessung. Die Verstellung erfolgt in Schritten unterschiedlicher Größe, um eine entsprechende Auflösung zu erreichen.

Vorteilhaft ist eine Messeinrichtung vorgesehen, mit der die Leistung des Prüfmotors ermittelt werden kann.

Die Leistungsermittlung kann direkt oder indirekt erfolgen. Bei der direkten Leistungsermittlung wird die Welle des Prüfmotors mit einem Drehmomentaufnehmer verbunden. Aus den ermittelten Messwerten lässt sich die Leistung für jeden Einspritzbeginn bestimmen. Bei einem vorteilhaften Ausführungsbeispiel wird der Prüfmotor mit einem Generator verbunden und die Messeinrichtung ermittelt die elektrische Leistung des Generators. Auch hier wird für jeden Einspritzbeginn die Leistung ermittelt.

Besonders vorteilhaft ist ein Speicher vorgesehen, in dem der Rechner die zu jedem eingestellten Einspritzbeginn ermittelte Leistung zusammen mit dem eingestellten Einspritzbeginn ablegt. Nach dem Testlauf kann auf diese Weise aus den im Speicher abgelegten Leistungen die höchste Leistung herausgesucht und der dazugehörige Einspritzbeginn ermittelt werden. Über diesen Einspritzbeginn ergibt sich dann aus der Kalibrierkurve die Cetanzahl des Kraftstoffs.

Weitere Einzelheiten und Vorteile der Erfindung ergeben sich aus den Unteransprüchen im Zusammenhang mit der Beschreibung eines Ausführungsbeispiels, das anhand der Zeichnung eingehend erläutert wird.

Es zeigt:
- Fig. 1: eine schematische Darstellung des Prüfaufbaus,
- Fig. 2: ein Ablaufdiagramm für den Kalibriervorgang,
- Fig. 3: ein Ablaufdiagramm für die Bestimmung der Zetahnzahl bei einem zu prüfenden Kraftstoff mit Validierung und
- Fig. 4: das Programmschema eines Testlaufs.

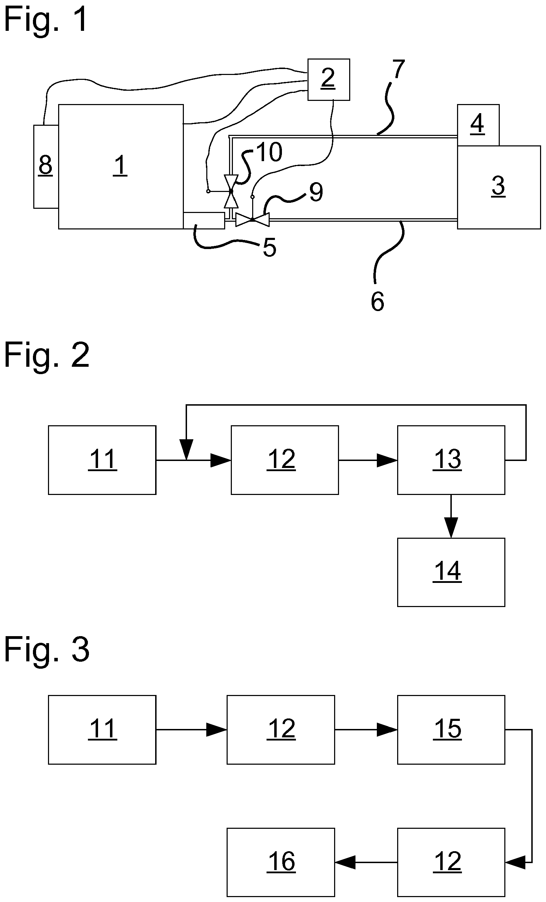

Der in Fig. 1 dargestellte Prüfaufbau zeigt den Prüfmotor 1. Bei diesem Motor handelt es sich um einen modernen Einzylinder-Dieselmotor mit Common-Rail-Technik. Die Motorsteuerung ist so gestaltet, dass der Einspritzbeginn während des Betriebs unabhängig von der Drehzahl und der Einspritzmenge verändert werden kann. Weiterhin ist ein Rechner 2 vorgesehen, über den der Ablauf der Prüfung manuell gesteuert werden kann. Es ist aber ebenso möglich, den Ablauf der Prüfung über ein Rechner-Programm vollautomatisch zu steuern. Der Tank 3 für den Prüfstoff mit bekannter Cetanzahl und der Tank 4 für den zu prüfenden Kraftstoff sind über die Kraftstoffleitungen 6 und 7 so mit dem Prüfmotor 1 verbunden, dass dieser sowohl mit dem Prüfstoff aus Tank 3 als auch mit dem zu prüfenden Kraftstoff aus Tank 4 versorgt werden kann. Zu diesem Zweck sind in den Kraftstoffleitungen 6 und 7 die Ventile 9 und 10 vorgesehen, die ebenfalls über den Rechner 2 steuerbar sind.

Die Kraftstoffheizung 5 soll sicherstellen, dass der dem Prüfmotor zugeführte Kraftstoff immer die gleiche Temperatur aufweist, so dass die Messergebnisse nicht von der Kraftstofftemperatur abhängig sind. Die Kraftstoffheizung 5 soll so ausgelegt werden, dass alle zu prüfenden Kraftstoffe eine Viskosität aufweisen, bei der keine Schwankungen der Einspritzmenge zu erwarten sind.

Die Kraftstoffheizung 5 ist möglichst nahe am Prüfmotor 1 vorgesehen. Die Zusammenführung verschiedener Kraftstoffleitungen findet direkt vor der Kraftstoffheizung 5 statt, so dass nur ein geringer Vorlauf notwendig ist und dass gewährleistet werden kann, dass sicher ein bestimmter Kraftstoff eingespritzt wird. Möchte man diesen Vorlauf weiter verkürzen, kann auch eine zweite Kraftstoffheizung vorgesehen werden, so dass die Zusammenführung der Kraftstoffe erst nach der Kraftstoffheizung direkt vor der Einspritzanlage geschieht.

Der Prüfmotor 1 weist auch einen Rücklauf auf, der in der Zeichnung aus Übersichtlichkeitsgründen nicht dargestellt ist. Dieser Rücklauf führt zu einem Abfalltank, der nach einer gewissen Betriebsdauer des Prüfmotors 1 eine Kraftstoffmischung aus bereit geprüften Kraftstoffen und aus Prüfstoffen enthält.

Soll mit den Kraftstoffen, insbesondere mit den Prüfstoffen sparsam umgegangen werden, kann zu jedem Kraftstoffbehälter zusätzlich ein eigener Rücklauf vorgesehen werden. Hierzu werden entsprechende Ventile in den Rücklaufleitungen vorgesehen. Bei dem Wechsel von einem ersten auf einen zweiten Kraftstoff wird dann zuerst das Ventil für den Rücklauf des ersten Kraftstoffs geschlossen und das Ventil für den Rücklauf in den Abfalltank geöffnet. Dieses Ventil bleibt so lange geöffnet bis sichergestellt ist, dass sich in der Einspritzanlage des Prüfmotors 1 kein Kraftstoffgemisch, sondern nur noch der zweite Kraftstoff befindet. Dann wird das Ventil für den Rücklauf in den Abfalltank geschlossen und das Ventil für den Rücklauf des zweiten Kraftstoffs geöffnet. Auf diese Weise kann sichergestellt werden, dass immer nur reiner Kraftstoff in den jeweiligen Tank rückgeführt wird.

Der Generator 8 wird über den Prüfmotor 1 angetrieben. In den Generator 8 ist eine Messeinrichtung integriert, mit der die erzeugte elektrische Leistung gemessen werden kann. Die Messeinrichtung übermittelt die gemessenen Werte an den Rechner 2.

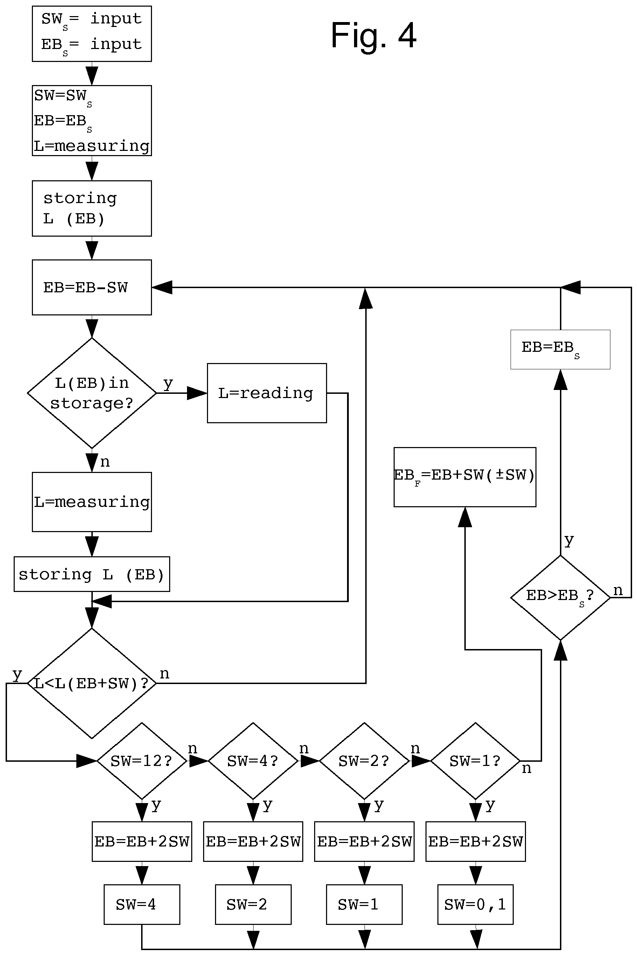

Eine Kalibrierung der Prüfanordnung ist mit relativ viel Aufwand verbunden, da eine größere Anzahl von Prüfstoffen mit bekannter Cetanzahl getestet werden muss. Hier ist für jeden Prüfstoff ein Testlauf notwendig, wie er in Fig. 4 aufgezeigt ist. Dieser in Fig. 4 gezeigte Testlauf ist auf ein Ergebnis mit hoher Auflösung und mit möglichst wenigen Messungen optimiert. Es wird dabei davon ausgegangen, dass die maximale Leistung eines jeden Kraftstoffs bei einem Einspritzbeginn zwischen 30° KW vor OT und 6° KW nach OT erreicht wird. Um einen unbekannten Kraftstoff zu testen, wird daher ein sechsstufiges Testverfahren vorgeschlagen.

Der in Fig. 2 gezeigte Kalibriervorgang beginnt mit dem Warmlaufen 11 des Prüfmotors 1. Wenn der Prüfmotor 1 seine Betriebstemperatur erreicht hat, wird ein Testlauf 12 (siehe Fig. 4) mit einem Prüfstoff mit bekannter Cetanzahl gestartet. Nach diesem Testlauf wird in Schritt 13 der Einspritzbeginn, für den die höchste Leistung ermittelt wurde, zusammen mit der bekannten Cetanzahl des Prüfstoffs abgespeichert. Danach wird ein anderer Prüfstoff mit der Kraftstoffleitung 7 verbunden und ein neuer Testlauf 12 wird gestartet. Es werden so viele Prüfstoffe geprüft, bis genügend Kalibrierpunkte zur Verfügung stehen, um daraus in Schritt 14 eine Kalibrierfunktion ermitteln zu können.

Eine Kalibrierkurve wird erstellt, indem jedem Einspritzbeginn mit maximaler Leistung die jeweils bekannte Cetanzahl des vermessenen Prüfstoffs zugeordnet wird. Aus dieser Kalibrierkurve lässt sich die Kalibrierfunktion entwickeln, mit der aus jedem Wert für den Einspritzbeginn mit maximaler Leistung die dazugehörige Cetanzahl abgeleitet werden kann. Die Ermittlung 14 und Abspeicherung der Kalibrierfunktion wird von dem Rechner 2 nach Abschluss der Kalibrierläufe durchgeführt.

In Fig. 3 ist schematisch die Vermessung eines unbekannten zu prüfenden Kraftstoffs mit einem bereits kalibrierten Prüfmotor 1 gezeigt. Auch hier muss der Prüfmotor 1 in einem ersten Schritt 11 warmlaufen. Dies erfolgt am besten mit dem Kraftstoff in dem größeren Tank 3, dessen Cetanzahl bei Anlieferung vermessen wurde und daher bereits bekannt ist. Die Ventile 9 und 10 werden entsprechend geschaltet. Es hat sich herausgestellt, dass der Prüfmotor 1 nach jeder Warmlaufphase validiert werden sollte. Zu diesem Zweck wird ein Testlauf 12 mit dem Prüfstoff mit bekannter Cetanzahl aus Tank 3 durchgeführt. Da hier sehr genau bekannt ist, bei welchem Einspritzbeginn mit der maximalen Leistung zu rechnen ist, kann der Testlauf 12 mit einer sehr kleinen Schrittweite nahe an dem erwarteten Wert für den Einspritzbeginn begonnen werden, so dass die Validierung nur kurze Zeit in Anspruch nehmen wird. Es kann z. B. 02° KW vor dem erwarteten Ergebnis mit der Messung begonnen und mit einer Schrittweite von 0,1° KW gemessen werden. Das Ergebnis dieses Testlaufs wird in Schritt 15 mit der bekannten Kalibrierkurve verglichen. Sollte eine Abweichung festgestellt werden, wird die Kalibrierfunktion entsprechend korrigiert und abgespeichert.

Nun werden über den Rechner 2 das Ventil 9 geschlossen und das Ventil 10 geöffnet, so dass der Prüfmotor jetzt mit dem zu prüfenden Kraftstoff aus Tank 4 versorgt wird. Es muss nun eine vorbestimmte Zeit gewartet werden bis sichergestellt ist, dass nur noch der zu prüfende Kraftstoff an der Verbrennung teilnimmt. Mit diesem zu prüfenden Kraftstoff wird jetzt ein neuer Testlauf 12 gestartet. Als Ergebnis des Testlaufs 12 erhält man einen Wert für den Einspritzbeginn bei dem der zu prüfende Kraftstoff die höchste Leistung des Prüfmotors 1 erzeugt. In Schritt 16 wird über die Kalibrierfunktion aus diesem Wert die Cetanzahl des zu prüfenden Kraftstoffs ermittelt.

Jeder Testlauf mit einem Kraftstoff - also sowohl mit einem Prüfstoff, als auch mit einem zu prüfenden Kraftstoff - setzt voraus, dass der Prüfmotor 1 bereits warmgelaufen ist und seine normale Betriebstemperatur erreicht hat. Direkt nach der Warmlaufphase wird die Einspritzmenge so eingeregelt, dass der Prüfmotor 1 eine vorbestimmte Drehzahl erreicht. Auch der Einspritzdruck wird auf einen vorbestimmten Wert, bei dem hier beschriebenen Prüfmotor auf 1600 bar, fixiert. Der Einspritzbeginn wird auf 30° KW vor OT eingestellt.

Die elektrische Leistung des Generators 8 wird kontinuierlich gemessen und an den Rechner 2 übermittelt. Sobald sich der Lauf stabilisiert hat, verändert sich die Leistung nur noch innerhalb einer vorbestimmten Bandbreite. Ist diese Veränderungsbandbreite erreicht speichert der Rechner den ermittelten Leistungswert in Relation zu dem eingestellten Einspritzbeginn ab.

Während der 1. Messreihe wird der Einspritzbeginn in 12°-Schritten verstellt, so dass eine sehr grobe Lokalisierung des gesuchten Einspritzbeginns mit der höchsten Leistung möglich ist. Ergibt sich beispielsweise für 18° KW vor OT eine niedrigere Leistung als als bei 30° KW vor OT so muss der Einspritzbeginn mit maximaler Leistung zwischen 30° und 18° KW vor OT liegen, da ein Einspritzbeginn von mehr als 30° KW vor OT praktisch nicht vorkommt. In diesem Fall sind lediglich 2 Messungen innerhalb der 1. Messreihe notwendig. Ist jedoch die Leistung bei 18° KW höher als bei 6° KW vor OT so kann der Einspritzbeginn mit der höchsten Leistung in dem Bereich zwischen 30° und 6° KW vor OT liegen.

Bei der 2. Messreihe wird nun in 4°-Schritten in Richtung OT verstellt. Bei jedem Einspritzbeginn wird wieder nach der Stabilisierungsphase der gemessene Leistungswert in Relation zu dem eingestellten Einspritzbeginn abgespeichert. Dieser Vorgang wird solange wiederholt bis ein Leistungswert gemessen wird, der geringer ist als derjenige, der bei dem letzten, zuvor eingestellten eingestellten Einspritzbeginn gemessen wurde. Bei dieser Messreihe kommt es im Höchstfall zu 4 Messungen, in dem o.g. Beispiel bei einem Einspritzbeginn von 26°, 22°, 14° und 10°, da die Leistungswerte bei 30°, 18° und 6° KW vor OT bereits existieren.

Wird der erste niedrigere Leistungswert beispielsweise bei einem Einspritzbeginn von 18° KW vor OT gemessen, muss das Maximum der erzielbaren Leistung bei einem Einspritzbeginn zwischen 26° und 18° vor OT liegen. Der genauer zu betrachtende Bereich kann so mit wenigen Messungen auf eine Breite von 8° eingeschränkt werden.

Bei der nächsten 3. Messreihe wird dieser in Frage kommende Bereich in kleineren Schritten abgetastet. Hierzu wird der vorletzte eingestellte Einspritzbeginn - in diesem Fall also 26° KW vor OT - um die neue Schrittweite verringert. Da die neue Schrittweite die Hälfte der alten Schrittweite, also 2° KW betragen soll, liegt der erste Punkt der zweiten Messreihe bei 24° KW vor OT. Bei dieser Messreihe müssen nun allerhöchstens zwei Messungen, nämlich bei 24° und bei 20° vor OT durchgeführt werden müssen, da die Werte für 26°, 22° und 18° vor OT bereits bei den vorhergehenden Messreihen abgespeichert worden sind.

Auch hier wird die Messreihe wiederum beendet, sobald ein gemessener oder abgespeicherter Wert geringer als der abgespeicherte Wert mit einem um 2° größeren Einspritzbeginn ist. Wird beispielsweise festgestellt, dass die bei 22° vor OT abgespeicherte Leistung geringer als die in dieser Messreihe gemessene Leistung bei 24° KW vor OT ist, befindet sich das Leistungsmaximum in dem Bereich zwischen 26° und 22° KW vor OT.

Es wird nun die 4. Messreihe mit einer Schrittweite von 1° KW gestartet. Die erste Messung wird - entsprechend dem Vorgehen bei der 3. Messreihe - bei 25° KW vor OT begonnen. Da die Leistungswerte für 26°, 24° und 22° KW vor OT bereits abgespeichert sind, müssen in der dritten Messreihe höchstens noch die Werte für 25° und 23 KW vor OT ermittelt werden. Beide Messungen müssen durchgeführt werden, wenn sich beispielsweise ergibt, dass ein Absinken des Leistungswerts erst von 23° nach 22° KW vor OT eintritt. In diesem Fall befindet sich das Leistungsmaximum bei einem Einspritzbeginn zwischen 24° und 22° KW vor OT.

Es wird nun eine 5. Messreihe mit einer Schrittweite von 0,5° KW durchgeführt. Diese Messreihe besteht wiederum höchstens aus zwei Messungen, nämlich bei einem Einspritzbeginn von 23,5° und 22,5° KW vor OT. Angenommen der Wert bei 22,5° KW vor OT ist erstmals kleiner gegenüber dem Wert bei einem Einspritzbeginn von 0,5° mehr, so befindet sich das Leistungsmaximum zwischen 23,5° und 22,5° KW vor OT.

Wenn die Genauigkeit der Messung auf ±0,1° KW festgelegt werden soll ist eine 6. Messreihe mit einer Schrittweite von 0,1° KW mit höchstens 8 Messungen erforderlich. Fällt folglich der Leistungswert zwischen 22,8° und 22,7° KW vor OT erstmals ab, so liegt das Maximum der Leistung bei diesem Prüfstoff bei 22,8 ±0,1° KW vor OT.

Als grundsätzliche Regel für alle Messreihen gilt, dass der erste Punkt einer Messreihe, bei dem der gemessene oder aufgezeichnete Leistungswert gegenüber dem davor liegenden Punkt absinkt, der Schlusspunkt dieser Messreihe ist. Bei der 1.-5. Messreihe gilt, dass der Anfangspunkt der neuen Messreihe als der Punkt mit dem Winkelwert des Schlusspunkts der alten Messreihe plus zwei mal der Schrittweite der alten Messreihe definiert wird. Im Höchstfall kann der Anfangspunkt einer Messreihe aber nur identisch mit dem Anfangspunkt der 1. Messreihe sein. Als Einspritzbeginn mit der maximalen Leistung wird der Schlusspunkt der 6. Messreihe plus die Schrittweite dieser Messreihe definiert. Die Genauigkeit liegt bei ±1 Schrittweiten-Intervall.

Geht man davon aus, dass sich das Maximum der Leistung bei einem Einspritzbeginn zwischen 30° KW vor OT und 6° KW nach OT einstellt, so sind für die Vermessung eines Kraftstoffs mit der oben angegebenen Genauigkeit folglich im Höchstfall 3 Messungen in der 1. Messreihe, 4 Messungen in der 2. Messreihe, jeweils 2 Messungen in der 3.-5. Messreihe und 8 Messungen in der 6. Messreihe, also insgesamt 21 Messungen erforderlich. Im besten Fall kommt man mit 6 Messungen aus. Man wird also davon ausgehen können, dass durchschnittlich 12-13 Messungen notwendig sind.

Es wurde herausgefunden, dass der Prüfmotor nach jeder Verstellung des Einspritzzeitpunktes 1-2 Minuten, im Schnitt also 1,5 Minuten benötigt um seinen Lauf zu stabilisieren. Die Zeit für einen kompletten Testlauf ergibt sich somit auf durchschnittlich weniger als 20 Minuten. Die Warmlaufzeit des Prüfmotors 1 ist hierbei nicht eingerechnet.

Es ist ersichtlich, dass sich die Zeit für einen Testlauf verringern lässt, wenn ungefähr bekannt ist, bei welchem Einspritzbeginn die maximale Leistung erreicht wird. In diesem Fall kann möglicherweise mit der zweiten Messreihe bei einem Einspritzbeginn von weniger als 26° KW vor OT begonnen und somit die erste Messreihe eingespart werden.

Dieses Vorgehen spielt insbesondere bei der Kalibrierung des Prüfmotors 1 eine Rolle wenn Prüfstoffe mit bekannter Cetanzahl vermessen werden. Hierzu muss nur bei einem Prüfstoff der vollständige Testlauf absolviert werden. Bei den restlichen Prüfstoffen, deren Cetanzahl und deren Abstand zu der Cetanzahl des bereits getesteten Prüfstoffs bekannt ist, kann relativ gut eingeschätzt werden in welchem Winkelbereich für den Einspritzbeginn die maximale Leistung liegen muss. Unter Umständen kann hier der Testlauf mit der 5. oder sogar der 6. Messreihe begonnen werden.

In Fig. 4 ist ein Schema für einen möglichen Programmablauf eines Testlaufs 12 des erfindungsgemäßen Verfahrens gezeigt. Das Programm arbeitet mit den folgenden Variablen:
- SW_{S}:: Startschrittweite, mit der der Testlauf begonnen werden soll
- SW:: Schrittweite
- EB_{S}:: Starteinspritzbeginn, mit dem der Testlauf begonnen werden soll
- EB:: Einspritzbeginn
- EB_{F}:: Final Einspritzbeginn, bei dem die maximale Leistung erreicht wird
- L:: Leistung

Die Variablen SW_{S} und EB_{S} werden abgefragt und von der Bedienperson festgelegt. Die einzugebenden Werte richten sich danach, wie genau der Einspritzbeginn mit der maximalen Leistung bereits vor dem Testlauf eingeschätzt werden kann. Zur Eingabe der Variablen SW_{S} werden nur die hinterlegten Daten angeboten.

Der eingegebene Einspritzbeginn wird nun an dem Prüfmotor eingestellt. Nach der Stabilisierung des Prüfmotors wird die erste Messung durchgeführt und die ermittelte Leistung in Abhängigkeit von dem eingestellten Einspritzbeginn abgespeichert.

Nach der Speicherung wird in die Variable EB der neue Einspritzbeginn eingelesen, in dem der alte Wert um die festgelegte Schrittweite verringert wird. Es wird nun geprüft, ob zu diesem Einspritzbeginn bereits ein gemessener Wert vorliegt. Ist dies der Fall, wird dieser Wert aus dem Speicher aus- und in die Variable L eingelesen.

Im anderen Fall wird dieser neue Einspritzbeginn an dem Prüfmotor eingestellt. Der Motor läuft nun für eine festgesetzte Zeit mit dieser Einstellung. Die Zeit ist so festgesetzt, dass eine Stabilisierung des Prüfmotors eintritt. Es kann aber auch in den Programmablauf noch eine Regelung eingebaut werden, mit der die Stabilisierung selbstständig erkannt wird. Bei so einer Regelung könnte eine Messschleife eingebaut werden, die so lange ausgeführt wird, bis sich die Messwerte nicht mehr über einen vorbestimmten Betrag hinaus verändern. Erst dann wird der letzte Messwert oder ein Mittelwert aus den letzten Messwerten in die Variable L eingelesen und wiederum in Abhängigkeit von dem eingestellten Einspritzbeginn abgespeichert.

Im nächsten Schritt wird geprüft, ob der letzte Messwert niedriger als der Messwert mit dem zuletzt eingestellten Einspritzbeginn ist. Ist das nicht der Fall, muss mit der selben Schrittweite weiter gemessen werden. Der Programmablauf geht hierzu in eine Schleife und springt zu dem Punkt zurück an dem in die Variable EB ein neuer Einspritzbeginn eingelesen wird, in dem der alte Wert um die Schrittweite in der Variablen SW verringert wird.

Diese Schleife wird so lange durchlaufen bis der letzte Messwert nicht mehr niedriger als der Messwert mit dem zuletzt eingestellten Einspritzbeginn ist. Jetzt wird die Schleife verlassen und im nächsten Schritt der Wert der Variablen SW abgefragt. Die Variable EB wird dann um zwei Schrittweiten aus der Variablen SW zurückgesetzt. Danach wird die Variable SW mit der nächst kleineren Schrittweite belegt.

Da es nicht sinnvoll ist mit der neuen Schrittweite die Messung bei einem Einspritzbeginn anzufangen, der weiter vor OT liegt als der Starteinspritzbeginn, wird geprüft, ob der Wert der Variablen EB größer als der Wert der Variablen EB_{S} ist. Ist dies der Fall wird der Wert der Variablen EB auf den Starteinspritzbeginn zurückgesetzt.

In jedem Fall ist jetzt die Variable EB mit einem Wert belegt, der nicht größer als der Starteinspritzbeginn ist. Es wird nun zu dem Punkt gesprungen, an dem in die Variable EB ein neuer Einspritzbeginn eingelesen wird, in dem der alte Wert um die Schrittweite in der Variablen SW verringert wird.

Ist die letzte Messreihe mit der Schrittweite 0,1 durchlaufen, wird der Punkt erreicht. an dem die Variable EBF belegt wird. In diese Variable wird nun ein Wert eingelesen der sich aus dem aktuellen Wert der Variablen EB plus dem aktuellen Wert aus der variablen SW ergibt. Die Genauigkeit dieses Ergebnisses beträgt ± dem aktuellen Wert aus der Variablen SW. In dem hier gezeigten Ausführungsbeispiel kann die Genauigkeit mit ± 0,1° KW angegeben werden.

### Bezugszeichenliste:

- 1: Prüfmotor
- 2: Rechner
- 3: Tank für den Prüfstoff mit bekannter Cetanzahl
- 4: Tank für den zu prüfenden Kraftstoff
- 5: Kraftstoffheizung
- 6: Leitung für den zu prüfenden Kraftstoff
- 7: Leitung für den Prüfstoff
- 8: Generator
- 9: Ventil für Prüfstoff
- 10: Ventil für zu prüfenden Kraftstoff
- 11: Warmlaufen
- 12: Testlauf entsprechend Fig. 4
- 13: Abspeichern der Kalibrierpunkte
- 14: Berechnung der Kalibrierfunktion
- 15: Korrektur der Kalibrierfunktion
- 16: Ermittlung der Cetanzahl

## Patentansprüche

1. Verfahren zum Ermitteln der Cetanzahl von Kraftstoffen mit einem Prüfmotor (1), wobei die Cetanzahl des zu prüfenden Kraftstoffs über einen Vergleich mit einer Reihe von Prüfstoffen mit bekannter Cetanzahl ermittelt wird, **dadurch gekennzeichnet, dass** für den zu prüfenden Kraftstoff der Einspritzbeginn ermittelt wird, bei dem der Prüfmotor die höchste Leistung erreicht, wobei der Prüfmotor (1) mit einer nicht unterbrochenen, kontinuierlichen Einspritzung betrieben wird.

2. Verfahren nach Anspruch 1, **dadurch gekennzeichnet, dass** die höchste Leistung bei unverändertem Einspritzdruck, unveränderter Einspritzmenge und gleicher Drehzahl ermittelt wird.

3. Verfahren nach einem der vorhergehenden Ansprüche, **dadurch gekennzeichnet, dass** bei einem Betrieb des Prüfmotors (1) mit den Prüfstoffen für jeden Prüfstoff der Einspritzbeginn bei der höchsten erreichten Leistung ermittelt wird.

4. Verfahren nach einem der vorhergehenden Ansprüche, **dadurch gekennzeichnet, dass** der Prüfmotor (1) mit dem zu prüfenden Kraftstoff betrieben wird, der Einspritzbeginn jeweils nach einer Stabilisierung des Prüfmotors (1) in vorbestimmten Schritten verändert wird, dass zu jedem eingestellten Einspritzbeginn die Leistung des Prüfmotors (1) ermittelt wird und dass zumindest die höchste erreichte Leistung zusammen mit dem dabei eingestellten Einspritzbeginn gespeichert wird.

5. Verfahren nach einem der vorhergehenden Ansprüche, **dadurch gekennzeichnet, dass** in vorbestimmten Abständen eine Validierung des Prüfmotors (1) durchgeführt wird.

6. Verfahren nach einem der vorhergehenden Ansprüche, **dadurch gekennzeichnet, dass** bei dem Betrieb mit einem Prüfstoff der Einspritzbeginn ermittelt wird, bei dem der Prüfmotors (1) die höchste Leistung erreicht und die Abweichung gegenüber einer früheren Kalibrierung oder Validierung auf die anderen Prüfstoffe übertragen wird.

7. Verfahren nach einem der vorhergehenden Ansprüche, **dadurch gekennzeichnet, dass** der Prüfmotor (1) wenigstens eine Hochdruckpumpe, ein Commonrail, einen Zylinder und eine Einspritzdüse aufweist und dass der Einspritzbeginn während des Betriebs des Prüfmotors (1) verstellbar ist.

8. Verfahren nach einem der vorhergehenden Ansprüche, **dadurch gekennzeichnet, dass** ein Rechner (2) vorgesehen ist, der den Einspritzbeginn in bestimmten Schritten verstellt.

9. Verfahren nach einem der vorhergehenden Ansprüche, **dadurch gekennzeichnet, dass** eine Messeinrichtung (2) vorgesehen ist, mit der die Leistung des Prüfmotors ermittelt werden kann.

10. Verfahren nach Anspruch 9, **dadurch gekennzeichnet, dass** der Prüfmotor (1) mit einem Generator (8) verbunden ist und die Messeinrichtung (2) die elektrische Leistung des Generators ermittelt.

11. Verfahren nach einem der Ansprüche 9 bis 10, **dadurch gekennzeichnet, dass** ein Speicher vorgesehen ist, in dem der Rechner (2) die zu jedem eingestellten Einspritzbeginn ermittelte Leistung zusammen mit dem eingestellten Einspritzbeginn ablegt.

## Claims

1. Method for determining the cetane number of fuels with a test engine (1), where the cetane number of the fuel to be tested is determined via a comparison with a series of test substances with a known cetane number, **characterized in that** the injection start is determined for the fuel to be tested where the test engine reaches the highest level of performance, wherein the test engine (1) is operated with an uninterrupted continuous injection.

2. Method according to claim 1, **characterized in that** the highest level of performance is determined at an unchanged injection pressure, an unchanged injection quantity and at the same rotational speed.

3. Method according to any one of the preceding claims, **characterized in that**, for each test substance, the injection start at the highest level of performance reached is determined during operation of the test engine (1) with the test substances.

4. Method according to any one of the preceding claims, **characterized in that** the test engine (1) is operated with the fuel to be tested, the injection start is changed after stabilizing the test engine (1) at predetermined intervals respectively, that the performance of the test engine (1) is determined at each configured injection start and that at least the highest level of performance reached is saved together with the injection start being configured in the process.

5. Method according to any one of the preceding claims, **characterized in that** a validation of the test engine (1) is carried out at predetermined intervals.

6. Method according to any one of the preceding claims, **characterized in that**, during operation with a test substance, the injection start is determined, where the test engine (1) has reached the highest level of performance and the deviation in relation to an earlier calibration or validation is transferred to the other test substances.

7. Method according to any one of the preceding claims, **characterized in that** the test engine (1) comprises at least one high-pressure pump, a common-rail system, a cylinder and an injector, and that the injection start can be adjusted during operation of the test engine (1).

8. Method according to any one of the preceding claims, **characterized in that** a computer (2) is provided that adjusts the injection start at certain intervals.

9. Method according to any one of the preceding claims, **characterized in that** a measuring apparatus (2) is provided, by means of which the level of performance of the test engine can be determined.

10. Method according to claim 9, **characterized in that** the test engine (1) is connected to a generator (8) and the measuring apparatus (2) determines the electrical power of the generator.

11. Method according to one of the claims 9 to 10, **characterized in that** a memory is provided in which the computer (2) stores the level of performance determined at each configured injection start along with the configured injection start.

## Revendications

1. Procédé pour déterminer l'indice de cétane de carburants avec un moteur d'essai (1), dans lequel l'indice de cétane du carburant à tester est déterminé par une comparaison avec une série de substances de test ayant un indice de cétane connu, **caractérisé en ce que** pour le carburant à tester est déterminé le début d'injection, dans lequel le moteur d'essai atteint l'injection maximale, dans lequel le moteur d'essai (1) est actionné avec une injection continue, non interrompue.

2. Procédé selon la revendication 1, **caractérisé en ce que** la puissance maximale est déterminée à une pression d'injection inchangée, une quantité d'injection inchangée et une vitesse de rotation semblable.

3. Procédé selon l'une des revendications précédentes, **caractérisé en ce que** lors d'un fonctionnement du moteur d'essai (1) avec les substances de test, pour chaque substance de test, le début d'injection est déterminé à la puissance maximale atteinte.

4. Procédé selon l'une des revendications précédentes, **caractérisé en ce que** le moteur d'essai (1) est actionné avec le carburant à tester, le début d'injection est modifié à chaque fois après une stabilisation du moteur d'essai (1) dans des étapes prédéterminées, **en ce qu'**à chaque début d'injection programmée, la puissance du moteur d'essai (1) est déterminée et **en ce qu'**au moins la puissance maximale atteinte est enregistrée conjointement avec le début d'injection programmé.

5. Procédé selon l'une des revendications précédentes, **caractérisé en ce qu'**à intervalles prédéterminés est effectuée une validation du moteur d'essai (1).

6. Procédé selon l'une des revendications précédentes, **caractérisé en ce que** lors du fonctionnement avec une substance d'essai est déterminé le début d'injection, dans lequel le moteur d'essai (1) atteint la puissance maximale et l'écart par rapport à un calibrage ou une validation antérieur(e) est transmis aux autres substances d'essai.

7. Procédé selon l'une des revendications précédentes, **caractérisé en ce que** le moteur d'essai (1) présente au moins une pompe haute pression, un Commonrail, un cylindre et une buse d'injection et **en ce que** le début d'injection est réglable pendant le fonctionnement du moteur d'essai (1).

8. Procédé selon l'une des revendications précédentes, **caractérisé en ce qu'**un ordinateur (2) est prévu, qui règle le début d'injection à certaines étapes.

9. Procédé selon l'une des revendications précédentes, caractérisé en qu'est prévu un dispositif de mesure (2) avec lequel peut être déterminée la puissance du moteur d'essai.

10. Procédé selon la revendication 9, **caractérisé en ce que** le moteur d'essai (1) est relié à un générateur (8) et le dispositif de mesure (2) détermine la puissance électrique du générateur.

11. Procédé selon l'une des revendications 9 à 10, **caractérisé en ce qu'**est prévue une mémoire, dans laquelle l'ordinateur (2) enregistre la puissance déterminée à chaque début d'injection programmé conjointement avec le début d'injection programmé.
